(19) **Europäisches Patentamt**

**European Patent Office**

**Office européen des brevets**

(11) **EP 1 477 801 B1**

(12) **EUROPÄISCHE PATENTSCHRIFT**

(45) Veröffentlichungstag und Bekanntmachung des Hinweises auf die Patenterteilung:
**14.09.2005  Patentblatt 2005/37**

(51) Int Cl.⁷: $G01N\ 33/00$, $G01N\ 1/22$, $G01M\ 15/00$

(21) Anmeldenummer: **03010792.4**

(22) Anmeldetag: **14.05.2003**

(54) **Verfahren und Vorrichtung zur Abgasmessung von Verbrennungskraftmaschinen**

Method and device for exhaust gas measurement of internal combustion engines

Procédé et dispositif de mesure de gaz d'échappement des moteurs à combustion interne

(84) Benannte Vertragsstaaten:
**AT BE BG CH CY CZ DE DK EE ES FI FR GB GR HU IE IT LI LU MC NL PT RO SE SI SK TR**

(43) Veröffentlichungstag der Anmeldung:
**17.11.2004  Patentblatt 2004/47**

(73) Patentinhaber: **Pierburg Instruments GmbH 41460 Neuss (DE)**

(72) Erfinder: **Schmidt, Roland 21244 Buchholz (DE)**

(74) Vertreter: **Grünberg, Thomas JUNG HML Schraudolphstrasse 3 80799 München (DE)**

(56) Entgegenhaltungen:
WO-A-96/01998          DE-A- 2 444 117
DE-A- 4 017 472        US-B2- 6 405 577

• **PATENT ABSTRACTS OF JAPAN vol. 004, no. 107 (P-021), 31. Juli 1980 (1980-07-31) & JP 55 065133 A (HORIBA LTD), 16. Mai 1980 (1980-05-16)**

**Beschreibung**

Die Erfindung betrifft ein Verfahren und eine Vorrichtung zur Abgasmessung von Verbrennungskraftmaschinen (Ottomotoren und Dieselmotoren) nach einem Vollstromverdünnungsverfahren, auch CVS-Verfahren (von Constant Volume Sampling) genannt, wobei das Verfahren bzw. die Vorrichtung sowohl für eine gravimetrische Abgaspartikelmessung als auch eine Emissionsmessung gasförmiger Abgasbestandteile geeignet sein soll.

Die Emission von gasförmigen und festen/flüssigen Schadstoffkomponenten im Abgas von Otto- und Dieselmotoren ist vom europäischen wie vom US-amerikanischen Gesetzgeber limitiert.

Nach den gängigen Vorschriften muss das Abgas eines Motors in einem sogenannten Vollstromverdünnungssystem (CVS-System - Constant Volume Sampling) verdünnt werden.

Die Messung der bei Dieselmotoren anfallenden partikulären Bestandteile erfolgt als gravimetrische Summenmessung über dem Versuchszeitraum, d.h. ein spezielles Partikelmessfilter wird vor und nach der Beladung mit verdünntem Abgas des Dieselmotors gewogen. Aus der Gewichtsdifferenz wird unter Berücksichtigung der gefahrenen Teststrecke bzw. der abgegebenen Arbeit die spezifische Partikelemission berechnet.

Bei Ottomotoren werden die gasförmigen Abgaskomponenten ebenfalls im verdünnten Abgas an einem CVS-System bestimmt. Hierbei wird das verdünnte Abgas in Sammelbeutel geleitet und nach Beendigung eines Testzyklus die Konzentration der einzelnen gasförmigen Abgaskomponenten im Sammelbeutel bestimmt.

Sowohl der Partikelgrenzwert als auch die Grenzwerte für gasförmige Schadstoffe wurden in den letzten Jahren drastisch abgesenkt. Dies hat zur Folge, dass sich der Unterschied zwischen dem Betrag der Messgrösse und der Messauflösung stetig verringert, wodurch die Messgenauigkeit abnimmt.

Hinsichtlich der einschlägigen europäischen Bestimmungen und damit verbundenen konkreten mess- und apparatetechnischen Anforderungen wird verwiesen auf: die Richtlinie 98/69/EG des Rates vom 13. Oktober 1998 sowie die Richtlinie 1999/96/EG des Europäischen Parlaments und des Rates vom 13. Dezember 1999 zur Angleichung der Rechtsvorschriften der Mitgliedstaaten über Massnahmen gegen die Emission gasförmiger Schadstoffe und luftverunreinigender Partikel aus Selbstzündungsmotoren zum Antrieb von Fahrzeugen und die Emission gasförmiger Schadstoffe aus mit Erdgas oder Flüssiggas betriebenen Fremdzündungsmotoren zum Antrieb von Fahrzeugen und zur Änderung der Richtlinie 88/77/EWG des Rates.

Bei einem Vollstromverdünnungssystem wird generell in einem Verdünnungstunnel der gesamte Abgasvolumenstrom eines Prüfmotors mit Verdünnungsluft so vermischt, dass der Gesamtvolumenstrom, der sich aus der Summe von Abgasvolumenstrom und Verdünnungsluftvolumenstrom zusammensetzt, konstant bleibt. Die Verdünnung des Abgases muss so gross gewählt werden, dass Wasserkondensation aus dem Abgas im Verdünnungstunnel und während der Probenahme vermieden, wobei bei Partikelemissionsmessung eine maximale Temperatur am Partikelfilter nicht überschritten werden darf.

Im System gilt die CVS-Bedingung:

$$\text{Abgasvolumenstrom} \cdot \text{Verdünnungsverhältnis} = \text{konst.}$$

bzw.

$$\text{Abgasvolumenstrom} + \text{Verdünnungsluftvolumenstrom} = \text{konst.}$$

Das Verdünnungsverhältnis VDV ist definiert als:

$$VDV = (\text{Verdünnungsluftstrom} + \text{Abgasvolumenstrom}) / \text{Abgasvolumenstrom}.$$

Aus der CVS-Bedingung ergibt sich ein hohes Verdünnungsverhältnis für Betriebspunkte mit einem niedrigen Abgasvolumenstrom und ein niedriges Verdünnungsverhältnis für Betriebspunkte mit einem hohen Abgasvolumenstrom.

Der Vorteil des CVS-Prinzips besteht vor allem darin, dass der Abgasvolumenstrom des Verbrennungsmotors variabel, aber der Gesamtvolumenstrom aus Abgas und Luft konstant ist, wodurch eine einfache Bestimmung der Partikelemission bzw. Belastung mit gasförmigen Schadstoffen möglich ist. Auf die dem Fachmann z.B. auch aus den oben zitierten Richtlinien hinlänglich bekannten Einzelheiten der Bestimmung wird nicht eingegangen.

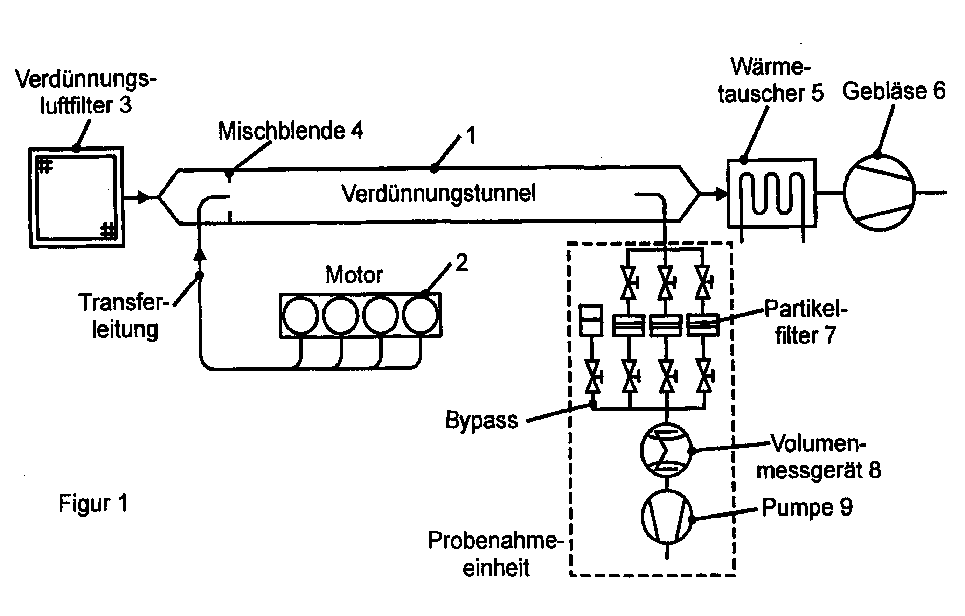

Ein konventionelles CVS-System gemäss Figur 1 für einen Dieselmotor 2 besteht beispielsweise aus den Einzelkomponenten Verdünnungsluftfilter 3, Verdünnungstunnel 1, Probenahmeeinheit und Absaugeinheit in Form eines Gebläses 6. Im Verdünnungstunnel 1 erfolgt die Vermischung von Raumluft und Abgas, wobei in der Regel eine Mischblende 4 eingesetzt wird, die die Durchmischung zusätzlich fördert. Am Ende des Tunnels befindet sich die Absaugeinheit, die entweder aus einer Verdrängerpumpe oder dem Gebläse 6 besteht, welches einen konstanten Volumenstrom fördert, oder z.B. aus einem Gebläse, dem gegebenenfalls eine oder mehrere Venturidüsen vorgeschaltet sind. Unabhängig vom Gebläse wird vor der Absaugeinheit ein Wärmetauscher 5 angeordnet, um für eine bessere Konstanz des Volumenstroms zu sorgen und das Gebläse zu schützen.

Die Probenahmeeinheit dient zur Sammlung der Partikel auf einem Filter 7. Dazu wird über eine Probenahmesonde ein definierter Teilstrom des verdünnten Abgases mit Hilfe der Probenahmepumpe aus dem Verdünnungstunnel 1 entnommen. Bei einer Partikelemissionsmessung wird der Teilstrom über einen Filterhalter geleitet, in dem sich Primär- und Sekundärpartikelfilter 7 befinden, und mit einem geeigneten Messgerät zur Volumen- bzw. Massenmessung aufgezeichnet. Der Teilstrom wird mit einer Pumpe 9 über einen dieser vorgeschalteten Mass Flow Controller 10 (Massendurchflussregler) gezogen. Für die hier nicht dargestellte Emissionsmessung gasförmiger Komponenten wird der Teilstrom in Beutel gepumpt.

Um die niedrigeren Emissionswerte der Motoren messtechnisch handhaben zu können, wurden verschiedenste Lösungen vorgeschlagen. Etliche der Lösungen richten sich allerdings auf Systeme mit Teilstromverdünnung wie z.B. die Verfahren der DE 41 21 928 A1 und US 6 112 575. Es wird in diesen bekannten Verfahren der Abgasvolumenstrom fortlaufend genau erfasst und versucht, die Verdünnung des Teilstroms so nachzuregeln, dass eine bessere Messgenauigkeit erzielt wird. Auch in dem von der US-amerikanischen Umweltbehörde EPA propagierten Bag Mini Diluter BMD muss der Abgasvolumenstrom zu jedem Zeitpunkt des Testzyklus bekannt sein. Bei diesem System wird das Verdünnungsverhältnis zu jedem Zeitpunkt des Testzyklus konstant gehalten, so dass der entnommene Teilstrom dem Abgasvolumenstrom ausreichend dynamisch nachgeregelt werden muss, um diese Konstanz für korrekte Messergebnisse zu gewährleisten.

Eine z.B. sekündliche Bestimmung des Abgasvolumenstroms während des Tests bei den Teilstromverdünnungssystemen ist problematisch, da der Abgasvolumenstrom im allgemeinen nur ungenau messbar ist, für eine Auswertung hinsichtlich Schadstoffemission in den im vorausgegangenen Abschnitt genannten Systemen aber zwingend notwendig ist.

In den CVS-Systemen besteht demgegenüber der Vorteil, dass die Ermittelung des Abgasvolumenstroms während des Tests nicht erforderlich ist. Wie oben bereits aufgezeigt, ändert sich bei den CVS-Systemen das Verdünnungsverhältnis im Verdünnungstunnel ständig, da der Abgasvolumenstrom kontinuierlich in einem Testzyklus verändert wird, der Gesamtvolumenstrom aber konstant bleibt. Da zur Vermeidung der Wasserkondensation das Verdünnungsverhältnis nach dem am grössten auftretenden Abgasvolumenstrom im Testzyklus ausgelegt werden muss, ergeben sich prinzipiell für niedrigere Abgasvolumenströme hohe Verdünnungsverhältnisse. Da femer ein konstanter Teilstrom des verdünnten Abgases entnommen wird, ergeben sich sehr niedrige Schadstoffkonzentrationen für die Messung mit Partikelfilter oder Sammelbeutel.

In der US 6 405 577 wird ein Vollstromverdünnungssystem beschrieben, in dem auf diese Problematik für die Bestimmung gasförmiger Schadstoffkonzentrationen eingegangen wird. Es werden mit variablen Venturidüsen sowohl der Gesamtvolumenstrom als auch der hieraus abgezweigte Probenahme- oder Teilstrom in Abhängigkeit vom Fahrzustand des jeweils getesteten Fahrzeugs verändert. Hierzu wird eine Steuereinheit verwendet, die die unterschiedlichen Fahrzustände des Testzyklusses und auch wahlweise von der Fahrzeugsteuerung direkt zugeführte Informationen über den aktuellen Fahrzustand berücksichtigt. Dabei wird der Fahrzustand in Phasen unterteilt, wobei in jeder Phase über die variable Venturidüse ein geeigneter konstanter Gesamtvolumenstrom eingestellt wird. Der Teilvolumenstrom wird so nachgeregelt, dass sich zum Gesamtvolumenstrom ein konstantes Verhältnis ergibt. Der Gesamtvolumenstrom wird mittels eines mit der variablen Venturidüse kombinierten Detektormechanismus ermittelt. Die Berechnung der jeweils aktuellen Strömungsrate des Gesamtvolumenstroms erfolgt anhand des Druckes und der Temperatur am Eingang der Gesamtvolumenstrom-Venturidüse sowie unter Heranziehung der aktuellen Einstellung der variablen Venturidüse. Hierzu sind Druck- und Temperatursensoren im besagten Detektormechanismus vorgesehen. Ferner wird für eine genaue Bestimmung der Schadstoffgaskonzentrationen die Strömungsrate des Abgases berechnet, indem die aktuelle Strömungsrate der dem System zugeführten Frischluft von der jeweils gerade vorliegenden Strömungsrate im Gesamtvolumenstrom subtrahiert wird. Die Venturidüse wird in Abhängigkeit der vorgegebenen Fahrzustände in einigen vorbestimmten Stufen gesteuert und der erzeugte Gesamtvolumenstrom aus den Düseneinstellwerten und detektierten Temperatur- und Druckwerten laufend berechnet und zur Nachstellung des Probenteilstroms herangezogen. Die Stufen, in denen der Gesamtvolumenstrom nach dem CVS-Verfahren jeweils auf einem konstanten Wert gehalten wird, werden so eingestellt, dass der Abgasvolumenstrom den Gesamtvolumenstrom an keiner Stelle übersteigt. In den einzelnen Stufen ist damit in der Regel das Verdünnungsverhältnis nicht gleich. Mit anderen Worten wird in diesem System ein Testzyklus entsprechend der Fahrzustände in Unterzyklen mit CVS-Bedingung auf unterschiedlichem Volumenströmungsniveau unterteilt.

Der Erfindung liegt die Aufgabe zugrunde, ein Verfahren und eine Vorrichtung zur Abgasmessung von Verbrennungskraftmaschinen (Ottomotoren und Dieselmotoren) nach einem Vollstromverdünnungsverfahren anzugeben, die sowohl für eine Partikelmessung als auch eine Konzentrationsmessung gasförmiger Emissionsbestandteile geeignet sind und mit geringem technischen Aufwand niedrige Schadstoffkonzentrationen bewältigen.

Diese Aufgabe wird durch den Gegenstand der unabhängigen Ansprüche gelöst. Bevorzugte Weiterbildungen sind in den Unteransprüchen definiert.

Erfindungsgemäss wird der Gesamtvolumenstrom im Gegensatz zu in der Praxis eingesetzten konventionellen Systemen nicht konstant gehalten. Es findet auch keine Unterteilung des jeweiligen Messzyklusses in CVS-Phasen unterschiedlichen Niveaus statt. Stattdessen wird der Gesamtvolumenstrom einem geglätteten Verlauf des Abgasstroms nachgeführt bzw. proportional nachgeregelt. Unter Abgasstrom wird der Abgasvolumenstrom und/oder der Abgasmassenstrom verstanden. Man kann wahlweise den einen und/oder anderen Abgasstromwert ermitteln, wobei man über die ideale Gasgleichung ohnehin einen gemessenen Abgasmassenstrom in den Abgasvolumenstrom umrechnen kann. Schliesslich ist es auch möglich, falls gewünscht, die Abgasvolumenstromnachführung durch eine Abgasmassenstromnachführung zu ersetzen. Der Verlauf des Gesamtvolumenstroms (Gesamtmassenstroms) zeigt somit den Verlauf des geglätteten Abgasstroms (Abgasvolumen- bzw. Abgasmassenstroms) in Abhängigkeit der Zeit und zwar in der Ordinate verschoben um ein jeweils gewähltes konstantes Verdünnungsverhältnis. Mit anderen Worten wird durch das gewünschte Verdünnungsverhältnis der Proportionalitätsfaktor der Nachregelung für einen Messzyklus vorgegeben.

Für die erfindungsgemässe Nachführung wird bevorzugt die Förderleistung einer Zieheinrichtung für den Gesamtvolumenstrom entsprechend nachgeregelt. Eine technisch einfache und daher bevorzugte Lösung besteht darin, die Drehzahl einer verwendeten Verdrängerpumpe, beispielsweise eines Rotationskolbengebläses nachzuregeln. Bevorzugt wird ein Roots-Gebläse eingesetzt. Durch diese kontinuierliche Nachführung wird das Verdünnungsverhältnis relativ konstant gehalten, nämlich bezogen auf den geglätteten Verlauf des Abgasstromes. Als nachteilig gegenüber dem System der US 6 405 577 könnte man werten, dass erfindungsgemäss eine Messung des Abgasstroms erforderlich ist, um dessen gemittelten Verlauf bestimmen zu können. Jedoch ist es erfindungsgemäss gerade nicht erforderlich, den Abgasvolumenstrom oder Abgasmassenstrom genau zu messen, so dass die Messung mit geringem zusätzlichen Aufwand durchführbar ist. Schliesslich ist es möglich, die Messung vorzugsweise einem eigentlichen Testzyklus vorzuschalten, wie weiter unten erläutert ist, so dass im Testzyklus selbst der Abgasstrom nicht mehr gemessen werden muss.

Zur Ermittelung des Abgasvolumenstromverlaufs kann man alternativ auch in einem solchen dem eigentlichen Testzyklus vorausgehenden Messzyklus den Abgasvolumenstrom kontinuierlich bei konstant gehaltenem Gesamtvolumenstrom aus diesem und dem angesaugten Luftvolumenstrom bestimmen. Hierzu kann man beispielsweise eine in konventionellen CVS-Systemen vorgesehene Einrichtung zur Messung der in den Verdünnungskanal angesaugten Luft nutzen. Voraussetzung für eine derartige Bestimmung des Abgasvolumenstromverlaufs ist, dass der Wert des Gesamtvolumenstroms so hoch eingestellt ist, dass das hierbei variable Verdünnungsverhältnis nicht zu gering wird. Im eigentlichen Testzyklus kann dann der geglättete Abgasstromverlauf eines solchen Messzyklusses zur erfindungsgemässen Nachführung des Gesamtvolumenstroms bei konstantem Verdünnungsverhältnis herangezogen werden.

Wird im erfindungsgemässen Verfahren das Verdünnungsverhältnis so klein wie möglich gewählt, um gerade die Wasserkondensation und ein Überschreiten der Filtertemperatur von 51,7°C zu vermeiden, können immer eine höhere gasförmige Schadstoffkonzentration im verdünnten Abgas bzw. eine höhere Partikelfilterbeladung und damit eine bessere Messgenauigkeit gewährleistet werden als bei Konstanthaltung des Gesamtvolumenstroms. Für die praktische Umsetzung bedeutet dies, das man für den jeweiligen Motor das optimale Verdünnungsverhältnis festlegt und dann mit diesem als Faktor der Nachführung den Gesamtvolumenstrom dem geglätteten Verlauf des Abgasstroms nachregelt.

Die Bezugsgrösse ist erfindungsgemäss nicht der Abgasvolumenstrom des Motors, sondern der Gesamtvolumenstrom aus Abgas und Verdünnungsluft. Diesem wird der Teilstrom des verdünnten Abgases zur Probenahme über ein Partikelfilter oder in den Sammelbeutel bevorzugt so nachgeregelt, dass ein konstantes Verhältnis dieser beiden Grössen während des Testzyklusses eingehalten wird.

Es gilt:

$$\text{Teilvolumenstrom/Gesamtvolumenstrom} = \text{konst.}$$

Dieser für einen Messzyklus konstante Verhältniswert kann z.B. für hohe und niedrige Schadstoffkonzentrationen angepasst werden, um die Messgenauigkeit noch weiter zu optimieren. So wird man bei kleiner Schadstoffkonzentration den Verhältniswert grösser machen.

Man könnte, obgleich mit den derzeitigen gesetzlichen Vorschriften nicht im Einklang, innerhalb des Messzyklusses den Verhältniswert zwischen Gesamt- und Teilvolumen auch definiert ändern und bei der Bestimmung des Emissionswertes entsprechend berücksichtigen. So wäre in der derzeitig vorgeschriebenen Formel für die Partikelemissionsbestimmung eine solche definierte Änderung des Teilvolumenstroms bereits berücksichtigbar.

Da der Gesamtvolumenstrom einem geglätteten Verlauf des Abgasvolumenstroms proportional nachgeregelt wird, braucht der Teilvolumenstrom aus dem verdünnten Abgas nur langsam geregelt zu werden. Die Schwierigkeiten einer dynamischen Messung von Teilvolumenstrom oder Gesamtvolumenstrom fallen weg. Der Gesamtvolumenstrom kann z. B. leicht aus der Drehzahl einer Absaugeinrichtung und deren Volumen bestimmt werden.

Um die Drehzahl und damit die Fördermenge der Absaugeinrichtung frei wählen zu können und innerhalb der dynamischen Grenzen der Absaugeinrichtung kontinuierlich verändern zu können, wird bevorzugt ein Roots-Gebläse eingesetzt, welches über einen Elektromotor angetrieben wird, der wiederum über einen Frequenzumrichter gesteuert wird. Dies stellt eine kostengünstige und technisch einfache Lösung dar. Prinzipiell ist es möglich, jede Art von Zieheinrichtung für den Gesamtvolumenstrom einzusetzen, mit der dieser entsprechend dem geglätteten Verlauf des Abgasvolumenstroms nachgeregelt werden kann. Da die Zieheinrichtung für den Teilvolumenstrom ebenfalls nicht hochdynamisch, sondern nur langsam nachzuregeln ist, ist auch diese Nachregelung mit geringem technischen Aufwand möglich. In konkreten Ausführungen wurden bei einer Emissionsmessung gasförmiger Bestandteile wegen der erforderlichen Dichtigkeit Membranvakuumpumpen und für Partikelmessung Drehschieberpumpen mit vorgeschaltetem Massenflussregler eingesetzt. Die Pumpen lassen sich alternativ auch ohne Massenflussregler nachführen. Schliesslich kann man, obgleich erfindungsgemäss nicht bevorzugt, sowohl für den Gesamt- als auch den Teilstrom Venturidüsen mit Verdrängerpumpen bzw. Gebläsen heranziehen, um die beiden Grössen proportional nachzuführen.

Die Anforderungen für die Regeldynamik sind weitestgehend frei wählbar. Zu berücksichtigen sind die Dynamik der Zieheinrichtung für den Teilstrom und des Gebläses für den Gesamtvolumenstrom. Der Gradient des geglätteten Verlaufs des Abgasvolumenstroms sollte einen vom jeweiligen System abhängigen maximalen Wert nicht übersteigen, der durch diese Dynamik noch geleistet werden kann. Je nach Ausführung der Zieheinrichtungen für den Teil- und Gesamtvolumenstrom und deren dynamischem Verhalten wird man sich an der Einrichtung mit dem schlechteren dynamischen Verhalten orientieren, wenn man den Glättungsalgorithmus vorgibt, so dass auch von dieser Einrichtung der gewünschte Verlauf nachgefahren werden kann.

Erfindungsgemäss werden in der Regel instationäre Testzyklen variierender Motorlast vorausgesetzt. Prinzipiell ist das erfindungsgemässe Verfahren jedoch auch auf Tests mit stationären Betriebspunkten anwendbar.

Im folgenden wird die Erfindung anhand der Zeichnungen näher erläutert. Es zeigen:

Figur 1 eine Prinzipdarstellung einer konventionellen CVS-Vorrichtung;

Figuren 2a und 2b eine Prinzipdarstellung eines Ausführungsbeispiels der erfindungsgemässen Vorrichtung für eine Emissionsmessung partikel- und gasförmiger Bestandteile; und

Figur 3 den Verlauf eines Beispiels für die Glättung eines Abgasvolumenstroms und entsprechende Einstellung des Gesamtvolumenstroms.

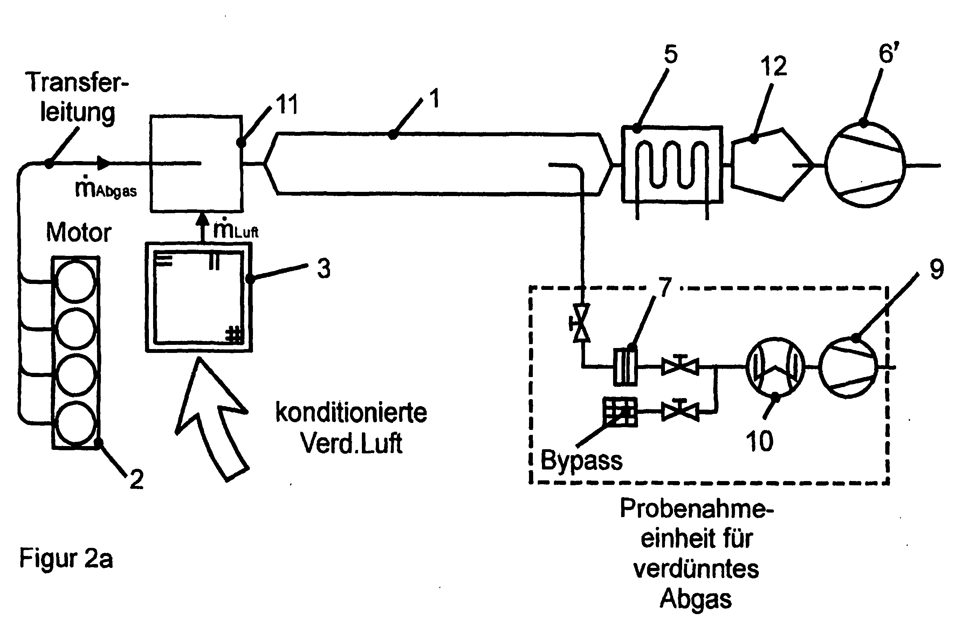

Das Abgas eines Dieselmotors 2 wird gemäss Figur 2a zunächst einer Mischkammer 11 zugeführt, in der es mit gefilterter Verdünnungsluft vermischt wird, um dann dem eigentlichen Verdünnungstunnel 1 zugeführt zu werden. Am Ende des Verdünnungstunnels wird ein Teilstrom des verdünnten Abgas-Luft-Gemisches in einer Probennahmeeinheit entnommen und über einen Filterhalter mit Hilfe einer Drehschieberpumpe 9 gezogen. In den Filterhalter werden z.B. teflonbeschichtete Glasfaserfilter 7 eingesetzt. Ein Massendurchflussregler 10 erlaubt die Nachführung des Teilvolumenstroms. Mit einem nicht dargestellten Drucksensor wird die Partikelbeladung des Filters 7 kontrolliert.

Das verdünnte Abgas-Luft-Gemisch durchläuft nach dem Verdünnungstunnel 1 und der Entnahmestelle für den Teilvolumenstrom einen Wärmetauscher 5, der es auf ca. 25°C herunterkühlt, bevor es durch einen Zyklon 12 zu einem Roots-Gebläse 6' gelangt. Der Zyklon 12 soll die Partikel aus dem Abgas-Luft-Gemisch entfernen, bevor diese in das Roots-Gebläse 6' gelangen könnten. Damit wird dieses effektiv vor Verschmutzung geschützt.

In Systemen mit Venturidüsen als Zieheinrichtung für Gesamt- und Teilvolumenstrom, die eine genaue Druck- und Temperaturmessung erfordern, liegt die Probenentnahmestelle für Emissionsmessungen gasförmiger Bestandteile im allgemeinen hinter dem Wärmetauscher. Hierin liegt auch ein prinzipieller Vorteil des Einsatzes eines Gebläses mit steuerbarer Drehzahl, für dessen Nachregelung weder Druck noch Temperatur genau zu messen sind.

Das Roots-Gebläse 6' wird mit einem nicht dargestellten Elektromotor betrieben und ist über einen Frequenzumrichter (nicht dargestellt) am Elektromotor drehzahlvariabel. Damit lassen sich erfindungsgemäss während eines Testzyklus die unterschiedlichen Gesamtvolumenströme für das verdünnte Abgas realisieren und damit relativ konstante Verdünnungsverhältnisse einstellen und einhalten. Die Zieheinrichtung für die Teilstromentnahme wird proportional den Änderungen im Gesamtvolumenstrom geregelt. Die Emission wird nach den einschlägigen Vorgaben des Gesetzgebers aus dem über die Dauer eines Testzyklusses integrierten Gesamtvolumen und Teilstromvolumen des verdünnten Abgases ermittelt.

In einer in die Praxis umgesetzten Vorrichtung wurden folgende Komponenten eingesetzt: Roots-Gebläse Delta Blower G M 15L der Aerzener Maschinenfabrik mit bis zu 4800 U/min, als Elektromotor für den Gebläseantrieb ein 4 kW Drehstromkäfigmotor von ABB Motors Oi, ein Frequenzumrichter ACS 601 von ABB Motors Oi, eine Drehschiebervakuumpumpe VCE 40 von Rietschle als Ziehpumpe für den Teilstrom bei Figur 2a zusammen mit einem Massenflussregler in Form eines Gasflussreglers 1559 von MKS Instruments. Abgesehen von einem Verdünnungsluftfilter der Firma Pierburg GmbH, waren die übrigen Komponenten wie Wärmetauscher, Verdünnungstunnel, Mischkammer und Zyklon von der Technischen Universität Darmstadt konzipiert worden.

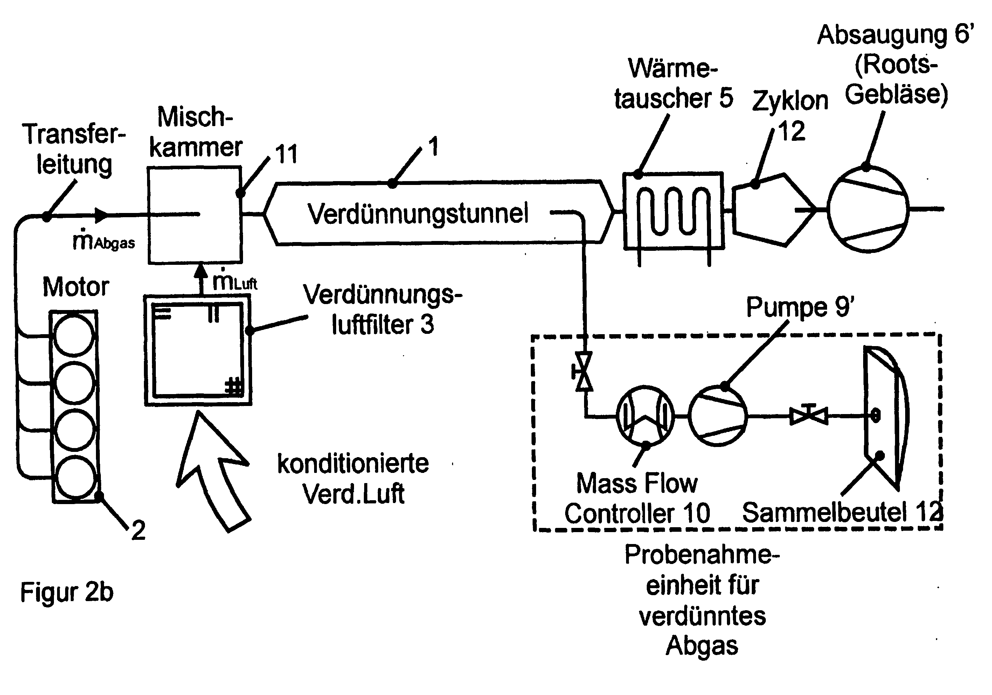

Figur 2b zeigt eine entsprechende mögliche erfindungsgemässe Vorrichtung für die Emissionsmessung gasförmiger Komponenten mit Hilfe eines Sammelbeutels 12. Bei der Pumpe ist hier wegen der erforderlichen Dichtheit z.B. eine Membranpumpe 9' eingesetzt. Die Pumpe 9' und der Massendurchflussregler 10 liegen vor dem Sammelbeutel 12.

Es gibt eine Vielzahl von Möglichkeiten, die Probenahmeeinheit anders auszulegen. Erfindungsgemäss muss lediglich sichergestellt werden, dass der gezogene Teilstrom in der gewünschten Weise nachgeregelt werden kann.

Eine bevorzugte Möglichkeit der Gewinnung des geglätteten Verlaufs vom Abgasvolumenstrom besteht darin, bei einem zu untersuchenden Versuchsmotor bzw. Versuchsfahrzeug zunächst den Abgasvolumenstrom z.B. sekündlich im jeweiligen Testzyklus aufzuzeichnen. Dabei ist infolge des erfindungsgemässen Prinzips eine exakte Messung nicht erforderlich. So können die Abgasmassenstromwerte durch sekündliche Bestimmung der Kraftstoffmasse mittels einer Kraftstoffwaage und Messung der gezogenen Luftmasse am Eingang des Motors ermittelt werden und hieraus über die ideale Gasgleichung der Abgasvolumenstromverlauf berechnet werden. Der Kraftstoffmassenstrom und der Luftmassenstrom werden hierzu addiert und vorhandene Laufzeit- bzw- Messzeitunterschiede korrigiert. Alternativ kann im Abgasstrang z.B. auch ein Messgerät vorgesehen werden, das auf Ultraschallmessung, Druckmessung oder anderen Verfahren beruht.

Bei einer sekündlichen Messung des Abgasvolumenstroms ist z.B. eine gleitende Mittelwertbildung über jeweils 10 Messwerte vernünftig, wobei durch eine nachfolgende Filterung mit einem FFT- Filter (Fast Fourier Transformation Filter) die noch verbliebenen schnellen Schwankungen aus der geglätteten Kurve herausgenommen werden. Bei abweichender Häufigkeit der Messungen wird das Fenster zur gleitenden Mittelwertbildung schmaler oder breiter gewählt. Die gleitende Mittelwertbildung mit nachfolgender Filterung in einem FFT-Filter oder auch z.B. einem Butterworth-Filter ist eine exakte Möglichkeit, den gemittelten Verlauf der gemessenen Werte zu gewinnen. Alternativ kann auch ein Filter mit geeignetem Glättungsalgorithmus herangezogen werden. Schliesslich ist es auch möglich, bereits während der Messung durch eine integrierende Messung eine Glättung des gemessenen Verlaufs vorzunehmen. Dabei ist allerdings ein Offset nicht vermeidbar, der je nach Messumfeld variiert, so dass der mathematische und messtechnische Aufwand vergleichsweise hoch sind. So wird erfindungsgemäss bevorzugt, vor einem eigentlichen Testzyklus mit Schadstoffemissionsermittelung zunächst in einem vorgeschalteten Testzyklus den gesamten Verlauf aufzunehmen und zu glätten und dann den geglätteten Verlauf im anschliessenden Test/Messzyklus zur proportionalen Regelung des Gesamtvolumenstroms heran zu ziehen. Dies hat sich bewährt. Man kann selbstverständlich zusätzlich auch während dieses Test/Messzykluses den Abgasvolumenstrom messen und glätten und die zuvor ermittelte Glättungskurve gegebenenfalls korrigieren. Bei den bisher gefahrenen Versuchen war dies allerdings nicht erforderlich und es reichte aus, für einen Testzyklus nur einmal vorab den Abgasvolumenstrom aufzuzeichnen und dessen geglätteten Verlauf für die nachfolgende(n) Messung bereitzustellen.

Die geglättete Kurve muss keineswegs eine genaue Wiedergabe des Abgasvolumenstroms darstellen. Kleine kurzzeitige Peaks des tatsächlich auftretenden Abgasvolumenstroms über dem geglätteten Verlauf, führen nicht notwendigerweise zu einer Wasserkondensation im verdünnten Abgas. Je nach der jeweiligen Neigung eines Motors, Verbrennungswasser zu erzeugen, wird man daher durch ein ausreichend hohes Verdünnnungsverhältnis einen der Ausprägung dieser Neigung angepassten Sicherheitsabstand vorgeben.

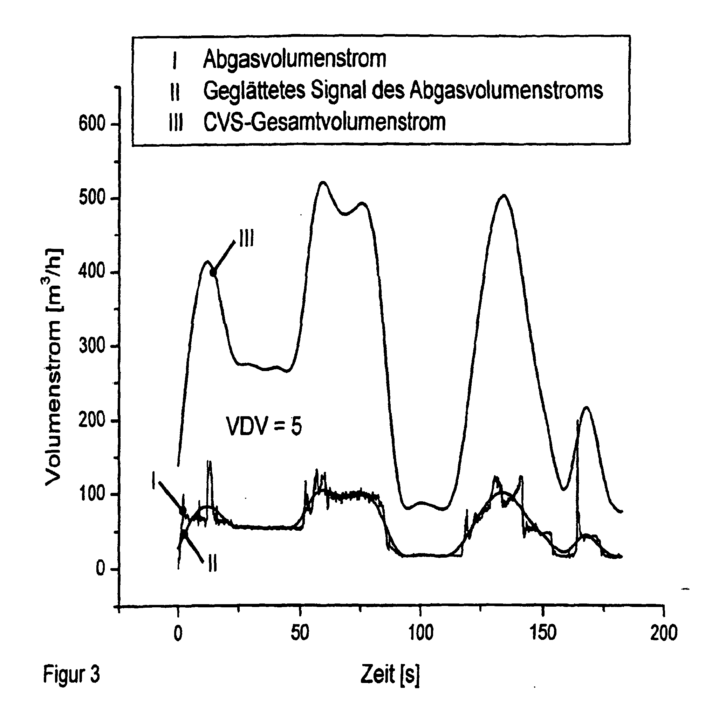

Um die Nachführung oder proportionale Nachregelung des Gesamtvolumenstroms zu realisieren, wurde eine Drehzahlsteuereinrichtung für das Roots-Gebläse folgendermassen vorgenommen. Der Abgasvolumenstrom wurde aus dem Abgasmassenstromverlauf, aufgenommen mit Luft- und Kraftstoffmassenstromwerten, berechnet. Es erfolgte eine gleitende Mittelwertbildung über jeweils 10 Sekunden und eine FFT-Filterung über 100 Werte. Das Ergebnis II dieser Glättung des Abgasstroms I ist in Figur 3 gezeigt. Der proportionale Gesamtvolumenstromverlauf III mit einem Verdünnungsverhältnis von 5 ist in Figur 3 ebenfalls eingezeichnet. Für diesen Verlauf wurden die entsprechend erforderlichen Drehzahlen ermittelt und über den Frequenzumrichter der Strom des Elektromotors des Roots-Gebläses entsprechend nachgefahren.

Sowohl für Partikelmessung als auch die Messung gasförmiger Bestandteile wurden die erfindungsgemäss ermittelten Werte mit den mittels einer konventionellen Vorrichtung gemäss Figur 1 gewonnenen Werten verglichen. Es wurde mit dem erfindungsgemässen Verfahren eine im Vergleich höhere Messgenauigkeit erzielt.

**Patentansprüche**

1.  Verfahren zur Abgasmessung von Verbrennungskraftmaschinen nach einem Vollstromverdünnungsverfahren, bei dem der Abgasvolumenstrom mit Verdünnungsluft so vermischt wird, dass in vorgegebenen Testzyklen ein definierter Gesamtvolumenstrom resultiert, aus dem ein Teilvolumenstrom abgezweigt wird, an dem eine Messung der Partikelemission und/oder gasförmiger Komponenten im Abgas vorgenommen wird, **dadurch gekennzeichnet, dass** ein geglätteter Verlauf (II) des in den Testzyklen variierenden Abgasstroms (I) ermittelt wird und der Gesamtvolumenstrom (III) den Änderungen im ermittelten geglätteten Verlauf des Abgasstroms proportional nachgeregelt wird.

2. Verfahren nach Anspruch 1, **dadurch gekennzeichnet, dass** die Nachregelung durch Regeln eines Rotationskolbengebläses, insbesondere Roots-Gebläses erfolgt, welches den Gesamtvolumenstrom zieht.

3. Verfahren nach Anspruch 1 oder 2, **dadurch gekennzeichnet, dass** der abgezweigte Teilstrom dem Gesamtvolumenstrom in einem konstanten Verhältnis von Teilstrom zu Gesamtvolumenstrom nachgeregelt wird.

4. Verfahren nach einem der vorhergehenden Ansprüche, **dadurch gekennzeichnet, dass** die Bestimmung des geglätteten Verlaufs des Abgasstroms in einem dem eigentlichen Testzyklus mit Emissionsmessung vorausgehenden Zyklus erfolgt und der darin gewonnene Verlauf für die proportionale Nachregelung im eigentlichen Testzyklus herangezogen wird.

5. Verfahren nach einem der vorhergehenden Ansprüche, **dadurch gekennzeichnet, dass** zur Bestimmung des geglätteten Verlaufs des Abgasstroms dieser aus der Kraftstoffmasse und Luftmasse am Motoreingang ermittelt wird.

6. Verfahren nach einem der vorhergehenden Ansprüche, **dadurch gekennzeichnet, dass** zur Bestimmung des geglätteten Verlaufs des Abgasstroms gemessene Abgasstromwerte des jeweiligen Testzyklus einer der folgenden Massnahmen unterzogen werden: gleitende Mittelwertbildung und Filterung, Filterung mit gleichzeitiger Glättung.

7. Verfahren nach einem der vorhergehenden Ansprüche, **dadurch gekennzeichnet, dass** die Gradienten der Glättung bei der Bildung des geglätteten Verlaufs des Abgasstroms unter Berücksichtigung der von Zieheinrichtungen für den Gesamtvolumenstrom und Teilvolumenstrom erbringbaren Gradienten in deren Regeldynamik auf maximale Werte begrenzt werden.

8. Vorrichtung zur Abgasmessung von Verbrennungskraftmaschinen nach einem Vollstromverdünnungsverfahren, mit einer Mischeinrichtung (11) zum Mischen des Abgasvolumenstroms mit Verdünnungsluft derart, dass in vorgegebenen Testzyklen ein definierter Gesamtvolumenstrom resultiert, mit Abzweigungsmitteln zum Abzweigen eines Teilvolumenstroms aus dem Gesamtvolumenstrom und einer Messeinrichtung (7,9,10; 7,9',10) zur Messung von Partikeln bzw. gasförmigen Konzentrationen im Abgas am abgezweigten Teilvolumenstrom, **gekennzeichnet durch** Mittel zum Messen des in den Testzyklen variierenden Abgasstroms, Mittel zum Glätten der gemessenen Werte des Abgasstroms und Mittel zum Einstellen einer Zieheinrichtung (6') für den Gesamtvolumenstrom derart, dass dieser dem geglätteter Verlauf des in den Testzyklen variierenden Abgasstroms proportional nachgeregelt wird.

9. Vorrichtung nach Anspruch 8, **dadurch gekennzeichnet, dass** als Zieheinrichtung für den Gesamtvolumenstrom ein Gebläse mit einstellbarer Drehzahl, insbesondere ein Roots-Gebläses (6') vorgesehen ist.

**Claims**

1. A method for measurement of exhaust gas of internal combustion machines according to a full-flow dilution method, in which the exhaust volume flow is mixed with dilution air so that a defined total volume flow results in predetermined test cycles from which total flow a partial volume flow is branched being subjected to measurement of particle emission and/or gaseous components in the exhaust gas, **characterized in that** a smoothed curve (II) of the exhaust flow (I) varying in said test cycles is determined and that the total volume flow (III) is subjected to a proportional control regulating it to the determined smoothed curve of the exhaust gas flow.

2. Method as recited in claim 1 **characterized in that** said regulation is accomplished by controlling a rotary piston blower, particularly a Roots blower, which draws the total volume flow.

3. Method as recited in Claim 1 or 2 **characterized in that** said branched partial flow is regulated in relation to the total volume flow in a constant ratio of the partial flow to the total volume flow.

4. Method as recited in any preceding claim **characterized in that** determining the smoothed curve of the exhaust flow is effected in a preliminary cycle that precedes the actual test cycle for the emissions measurement, and that the curve obtained thereby is utilized for the subsequent proportional control during the actual test cycle.

5. Method as recited in any preceding claim **characterized in that**, for determining the smoothed curve of the exhaust flow, the latter is determined from the fuel mass and the air mass at the motor inlet.

6. Method as recited in any preceding claim **characterized in that**, for determining the smoothed curve of the exhaust flow, measured exhaust flow values of the respective test cycle are subjected to one of the following steps: sliding averaging and filtering, filtering with simultaneous smoothing.

7. Method as recited in any preceding claim **characterized in that** the gradients of smoothing when determining the smoothed curve of the exhaust flow, are, taking into consideration possible gradients which can be afforded by drawing devices, limited to maximum values of the control dynamic of said possible gradients.

8. A device for measurement of exhaust gas of internal combustion machines according to a full-flow dilution method, having a mixing device (11) for mixing the exhaust volume flow with dilution air so that a defined total volume flow results in predetermined test cycles, branching means for branching a partial volume flow from the total volume flow and measurements means (7,9,10; 7, 9',10) for measuring particles or gas concentrations, respectively, in the exhaust gas of the branched partial volume flow,
**characterized by** means for measuring the exhaust flow varying in the test cycles, means for smoothing the measured values of the exhaust flow and means for adjusting means (6') for drawing the total volume flow such that the same is subjected to a proportional control regulating it to the smoothed curve of the exhaust flow varying in said test cycles.

9. Device as recited in claim 8, **characterized in that** blowing means with adjustable rotational speed, particularly a Roots blower (6'), are provided as means for drawing the total volume flow.

**Revendications**

1. Procédé de mesure de gaz d'échappement de moteurs à combustion interne selon un procédé de dilution complète du flux, dans lequel le volume de flux de gaz d'échappement est mélangé avec de l'air d'appoint de manière que dans des cycles de test prédéfinis, un volume total de flux défini en résulte duquel un volume partiel de flux est dérivé, dans lequel une mesure de l'émission de particules et/ou de composants gazeux dans le gaz d'échappement est opérée, **caractérisé en ce qu'**une évolution lissée (II) du flux de gaz d'échappement (I) variant dans les cycles de test est déterminée et le volume total de flux (III) est réajusté proportionnellement aux variations dans l'évolution lissée déterminée du flux de gaz d'échappement.

2. Procédé selon la revendication 1, **caractérisé en ce que** le réajustement s'effectue via le réglage d'un compresseur à piston rotatif, en particulier un compresseur Roots, lequel tire le volume total de flux.

3. Procédé selon la revendication 1 ou 2, **caractérisé en ce que** le flux partiel dérivé est réajusté par rapport au volume total de flux dans une proportion constante de flux partiel par rapport au volume total de flux.

4. Procédé selon l'une quelconque des revendications précédentes, **caractérisé en ce que** la détermination de l'évolution lissée du flux de gaz d'échappement s'effectue dans un cycle antérieur au cycle de test proprement dit avec mesure d'émission et l'évolution ainsi obtenue est mise à contribution pour le réajustement proportionnel dans le cycle de test proprement dit.

5. Procédé selon l'une quelconque des revendications précédentes, **caractérisé en ce qu'**en vue de la définition de l'évolution lissée du flux de gaz d'échappement, ce dernier est déterminé à partir de la masse de carburant et de la masse d'air à l'entrée du moteur.

6. Procédé selon l'une quelconque des revendications précédentes, **caractérisé en ce qu'**en vue de la définition de l'évolution lissée du flux de gaz d'échappement, des valeurs de flux de gaz d'échappement mesurées du cycle de test correspondant sont soumises à une des mesures suivantes : formation de la moyenne mobile et filtrage, filtrage avec lissage simultané.

**7.** Procédé selon l'une quelconque des revendications précédentes, **caractérisé en ce que** les gradients du lissage sont limités à des valeurs maximum lors de la formation de l'évolution lissée du flux de gaz d'échappement en tenant compte des gradients pouvant être fournis par des dispositifs de tirage pour le volume total de flux et le volume partiel de flux dans leur dynamique normale.

**8.** Procédé de mesure de gaz d'échappement de moteurs à combustion interne selon un procédé de dilution complète du flux, avec un dispositif mélangeur (11) destiné à mélanger le volume de flux de gaz d'échappement avec de l'air d'appoint de manière que dans des cycles de test prédéfinis, un volume total de flux défini en résulte, avec des moyens de dérivation destinés à dériver un volume partiel de flux du volume total de flux et un dispositif de mesure (7, 9, 10 ; 7, 9', 10) destiné à la mesure de particules et/ou de concentrations gazeuses dans le gaz d'échappement dans le volume partiel de flux dérivé,

   **caractérisé par** des moyens destinés à mesurer le flux de gaz d'échappement variant dans les cycles de test, des moyens destinés à lisser les valeurs mesurées du flux de gaz d'échappement et des moyens destinés à régler un dispositif de tirage (6') pour le volume total de flux de telle manière que ce dernier est réajusté proportionnellement à l'évolution lissée du flux de gaz d'échappement variant dans les cycles de test.

**9.** Procédé selon la revendication 8, **caractérisé en ce qu'**un compresseur à régime réglable, en particulier un compresseur Roots (6'), est prévu en tant que dispositif de tirage pour le volume total de flux.

Figur 1

Verdünnungs-luftfilter 3

Mischblende 4

1

Verdünnungstunnel

Wärme-tauscher 5

Gebläse 6

Transfer-leitung

Motor

2

Partikel-filter 7

Bypass

Volumen-messgerät 8

Pumpe 9

Probenahme-einheit

EP 1 477 801 B1

6'

12

5

1

11

9

10

7

Bypass

Probenahmeeinheit für
verdünntes
Abgas

konditionierte
Verd.Luft

3

2

Transfer-
leitung

$\dot{m}_{Abgas}$

$\dot{m}_{Luft}$

Motor

Figur 2a

11

Absaugung 6' (Roots-Gebläse)

Zyklon 12

Wärme-tauscher 5

Pumpe 9'

Sammelbeutel 12

Mass Flow Controller 10

Probenahme-einheit für verdünntes Abgas

Verdünnungstunnel

1

Verdünnungs-luftfilter 3

konditionierte Verd.Luft

Misch-kammer

11

$\dot{m}_{Luft}$

Transfer-leitung

$\dot{m}_{Abgas}$

Motor

2

Figur 2b

Figur 3